# EUROPEAN PATENT SPECIFICATION

(11) **EP 2 619 579 B1**
(45) Date of publication and mention of the grant of the patent: **07.12.2016**
(21) Application number: 11827399.4
(22) Date of filing: 21.09.2011
(51) Int. Cl.: G01N 33/53, G01N 33/68, G01N 33/74, G01N 33/564

(54) **DIAGNOSTIC TESTS FOR IMMUNE REACTIVITY WITH HUMAN ENDOTHELIAL CELL GROWTH FACTOR**
DIAGNOSTISCHE IMMUNREAKTIVITÄTSTESTS MIT DEM MENSCHLICHEN ENDOTHELZELLENWACHSTUMSFAKTOR
TESTS DE DIAGNOSTIC UTILISANT LA RÉACTIVITÉ IMMUNITAIRE AVEC LE FACTEUR DE CROISSANCE DÉRIVÉ DE CELLULES ENDOTHÉLIALES HUMAINES

(30) Priority: 21.09.2010 US 384733 P
(43) Date of publication of application: 31.07.2013
(73) Proprietor: The General Hospital Corporation, Boston, MA 02114 (US); Trustees of Boston University, Boston, MA 02215 (US)
(72) Inventor: STEERE, Allen C., Wellesley, MA 02481 (US); DROUIN, Elise, Stoneham, MA 02180 (US); COSTELLO, Catherine, Reading, MA 01867 (US); SEWARD, Robert, Malden, MA 02148 (US)
(74) Representative: Sweetinburgh, Mark Roger
(86) International application number: PCT/US2011/052456
(87) International publication number: WO 2012/040264

(56) References cited:
- US-A1- 2004 137 514
- US-A1- 2007 148 704
- D. M. GROSS: "Identification of LFA-1 as a Candidate Autoantigen in Treatment-Resistant Lyme Arthritis", SCIENCE, vol. 281, no. 5377, 31 July 1998 (1998-07-31), pages 703-706, XP055104731, DOI: 10.1126/science.281.5377.703
- SLAGER ELISABETH H ET AL: "Identification of the angiogenic endothelial-cell growth factor-1/thymidine phosphorylase as a potential target for immunotherapy of cancer", BLOOD, AMERICAN SOCIETY OF HEMATOLOGY, US, vol. 107, no. 12, 15 June 2006 (2006-06-15), pages 4954-4960, XP002475032, ISSN: 0006-4971, DOI: 10.1182/BLOOD-2005-09-3883
- MASANORI TAKEUCHI ET AL: "Aberrant production of gliostatin/platelet-derived endothelial cell growth factor in rheumatoid synovium", ARTHRITIS & RHEUMATISM, vol. 37, no. 5, 1 May 1994 (1994-05-01), pages 662-672, XP055104142, ISSN: 0004-3591, DOI: 10.1002/art.1780370509
- SHINSUKE SAITO ET AL: "Expression of platelet-derived endothelial cell growth factor correlates with good prognosis in patients with colorectal carcinoma", CANCER, vol. 88, no. 1, 1 January 2000 (2000-01-01), pages 42-49, XP055103705, ISSN: 0008-543X, DOI: 10.1002/(SICI)1097-0142(20000101)88:1<42:: AID-CNCR7>3.0.CO;2-M
- SHIQIAN SHEN ET AL: "Treg cell numbers and function in patients with antibiotic-refractory or antibiotic-responsive lyme arthritis", ARTHRITIS & RHEUMATISM, vol. 62, no. 7, 26 March 2010 (2010-03-26) , pages 2127-2137, XP055096804, ISSN: 0004-3591, DOI: 10.1002/art.27468
- ALLEN C STEERE ET AL: "Antibiotic-refractory Lyme arthritis is associated with HLA-DR molecules that bind a Borrelia burgdorferi peptide", THE JOURNAL OF EXPERIMENTAL MEDICINE, ROCKEFELLER UNIVERSITY PRESS, US, vol. 203, no. 4, 17 April 2006 (2006-04-17), pages 961-971, XP002632126, ISSN: 0022-1007, DOI: 10.1084/JEM.20052471
- ELISE E. DROUIN ET AL: "A novel human autoantigen, endothelial cell growth factor, is a target of T and B cell responses in patients with Lyme disease", ARTHRITIS & RHEUMATISM, vol. 65, no. 1, 27 December 2012 (2012-12-27), pages 186-196, XP055104144, ISSN: 0004-3591, DOI: 10.1002/art.37732
- SLAGER E.H. ET AL.: 'Identification of the angiogenic endothelial-cell growth factor-1/thymidine phosphorylase as a potential target for immunotherapy of cancer' BLOOD vol. 107, no. 12, 15 June 2006, pages 4954 - 4960, XP002475032
- SHEN S. ET AL.: 'TREG CELL NUMBERS AND FUNCTION IN PATIENTS WITH ANTIBIOTIC -REFRACTORY OR ANTIBIOTIC RESPONSIVE LYME ARTHRITIS' ARTHRITIS & RHEUMATISM vol. 62, no. 7, 31 July 2010, pages 2127 - 2137, XP055096804
- STEERE A.C. ET AL.: 'ANTIBIOTIC-REFRACTORY LYME ARTHRITIS IS ASSOCIATED WI TH HLA-DR MOLECULES THAT BIND A BORRELIA BURGDORFERI PEPTIDE' THE JOURNAL OF EXPERIMENTAL MEDICINE vol. 203, no. 4, 17 April 2006, pages 961 - 971, XP002632126

## Description

### FIELD OF THE INVENTION

The present invention provides for methods and kits for diagnosing Lyme disease-associated arthritis.

### BACKGROUND

Lyme disease, or borreliosis, is the most common vector-borne infectious disease in North America, Europe and Asia. Lyme disease has major public health and economic effects: the estimated annual cost is approximately $1 billion in the U.S. alone. State health departments reported about 30,000 confirmed cases and 8,500 probable cases of Lyme disease to the Centers for Disease Control and Prevention in 2009, representing a 3.6 percent increase in confirmed cases compared to the previous year.

Caused by the spirochete bacterium *Borrelia,* and transmitted to humans through the bite of infected ticks, Lyme disease is a multi-system disorder that is treatable with antibiotics and can affect the nervous system, heart, and in particular the joints. Some patients develop chronic Lyme disease, a condition characterized by persistent musculoskeletal and peripheral nerve pain, fatigue, and memory impairment. In subjects with joint involvement, a small percentage develop proliferative synovitis that persists for months or several years after apparent spirochetal killing with antibiotics, referred to as antibiotic-refractory arthritis. This disease course is hypothesized to result from infection-induced autoimmunity. D. M. GROSS et al. (1998) discloses that patients with treatment resistant Lyme arthritis respond to human leucocyte function-associated antigen-1 (hLFA-1), as was determined using ELISPOT IFN-γ read out upon stimulation of synovial fluid T-cells with hLFA-1. However, none of the previously proposed candidate autoantigens induced substantial T- and B-cell responses in antibiotic-refractory Lyme arthritis patients. Hence, there remains a need in the art for characterization of the autoantigen(s) implicated in Lyme arthritis patients.

### SUMMARY

The embodiments of the present invention provide for novel biomarker antigen(s), platelet-derived endothelial cell growth factor (ECGF), useful for diagnosing Lyme arthritis. More specifically, tandem mass spectrometry identified HLA-DR self-peptides presented *in vivo* from subjects' synovial tissue, the target tissue in this disease. Of 120 peptides identified from one patient, one peptide, which originated from the source protein platelet-derived endothelial cell growth factor (ECGF), induced peripheral blood mononuclear cells (PBMC) to proliferate and secrete IFN-γ *in vitro*. It was then shown that many patients with antibiotic-refractory arthritis had T-cell responses to ECGF peptides and autoantibodies to this self protein. Furthermore, the majority of patients in the refractory group had greater amounts of ECGF in joint fluid and synovial tissue, where it could be presented by HLA-DR molecules on local antigen-presenting cell. In addition, IgG anti-ECGF antibodies were found predominately in Lyme disease, and were present significantly more often in those subjects with antibiotic-refractory arthritis. Thus, ECGF is the first antigen identified that induces both T- and B-cell responses in subjects with antibiotic-refractory Lyme arthritis. The methods and compositions of the present embodiments should aid clinicians in designing better therapeutic approaches in treating subjects suffering from chronic inflammatory arthritis. Moreover, the present methodology, that combines discovery-based proteomics and translational research, is applicable to other autoimmune diseases where identifying pathogenic autoimmune responses has been a difficult challenge.

An embodiment of the present invention provides for a method for diagnosing Lyme arthritis in a subject, the method comprising analysing a biological sample obtained from a subject having Lyme disease for the presence of endothelial cell growth factor (ECGF) autoantibody by contacting said biological sample with an immunoassay comprising an ECGF antigen and wherein said biological sample is obtained from peripheral blood serum, synovial fluid, synovial tissue, peripheral blood mononuclear cells (PBMC), or synovial fluid mononuclear cells (SFMC). The immunoassay may be an ELISA, agglutination test, direct immunofluorescence assay, indirect immunofluorescence assay, western blot, an immunoblot assay, and the like. Alternatively, the immunoassay may be a T-cell proliferation assay such as ³H-thymidine incorporation, CFSE dilution, etc. The immunoassay may be a T-cell reactivity assay; for example, an immunoassay that comprises measuring secretion of IFN-γ from individual cells, e.g., an ELISpot immunoassay. In particular, the ECGF antigen used for such assays may consist of the intact protein or specific ECGF peptides. These peptides may include at least one ECGF peptide selected from the group consisting of:
LGRFERMLAAQGVDPG (SEQ ID NO: 1); ADIRGFVAAVVNGSAQGAQI (SEQ ID NO:2); DKVSLVLAPALAACG (SEQ ID NO:3); SKKLVEGLSALVVDV (SEQ ID NO:4); KTLVGVGASLGLRVAAALTAMD (SEQ ID NO:5); LRDLVTTLGGALLWL (SEQ ID NO:6); GTVELVRALPLALVLH (SEQ ID NO:7). The biological sample may be obtained from a subject suffering from chronic, inflammatory arthritis. In another aspect, a positive result of immunoreactivity of the biological sample with the ECGF antigen is indicative of Lyme arthritis, particularly antibiotic-refractory Lyme arthritis.

Another embodiment of the invention provides for the use of an isolated ECGF antigen as a biomarker for diagnosing Lyme arthritis. The isolated ECGF antigen can be human ECGF intact protein or specific ECGF peptides, or may include at least one of the following peptides: LGRFERMLAAQGVDPG (SEQ ID NO: 1); ADIRGFVAAVVNGSAQGAQI (SEQ ID NO:2); DKVSLVLAPALAACG (SEQ ID NO:3); SKKLVEGLSALVVDV (SEQ ID NO:4); KTLVGVGASLGLRVAAALTAMD (SEQ ID NO:5); LRDLVTTLGGALLWL (SEQ ID NO:6); GTVELVRALPLALVLH (SEQ ID NO:7).

Also described therein is a method of identifying antigens associated with autoimmune disorders. A biological sample is obtained from the subject, for example, blood, synovial fluid, or synovial tissue; HLA-DR-presented peptides are eluted from the sample; eluted peptides are identified, for example by mass spectrometry; corresponding peptides are synthesized, and; synthesized peptides are reacted with a biological sample obtained from the same subject, for example peripheral blood or synovial fluid mononuclear cells; whereby the *in vitro* reaction (i.e., a change in the sample) is characterized as an indicator of disease state.

A specific embodiment provides for a method for determining whether a subject suffering from chronic inflammatory arthritis associated with Lyme disease bears T-cells reactive to ECGF or ECGF peptides by (a) providing a set of synthesized ECGF peptides that are predicted to be presented by HLA-DR molecules; (b) stimulating peripheral blood mononuclear cells (PBMC) or the synovial fluid mononuclear cells (SFMC) obtained from the subject with one of said ECGF peptides; and (c) measuring T-cell proliferation *in vitro* or secretion of IFN-γ as a test for T-cell reactivity. The subject can be suffering from either antibiotic-refractory or antibiotic-responsive Lyme arthritis.

Another specific embodiment provides for a method for determining whether a subject suffering from chronic inflammatory arthritis associated with Lyme disease contains a B-cell response to ECGF resulting in the production of autoantibodies found in serum or synovial fluid against ECGF, comprising the steps of (a) providing an isolated antigen, wherein said antigen is ECGF, or an ECGF peptide, (b) providing a serum sample from a subject; (c) conducting an immunoassay on said biological sample utilizing said antigen, wherein said immunoassay detects the presence of antibodies that recognize said ECGF; and (d) determining that the subject contains an antibody against said antigen if the results of the immunoassay indicate that an antibody that recognizes said antigen is present in said biological sample. The subject can be suffering from either antibiotic-refractory or antibiotic-responsive Lyme arthritis. The immunoassay may comprise an ELISA, agglutination test, direct immunofluorescence assay, indirect immunofluorescence assay, western blot, an immunoblot assay, and the like.

Yet another embodiment is directed to a kit for identifying a subject with Lyme arthritis, comprising ECGF or a set of synthesized ECGF peptides, and reagents necessary for conducting an immunoassay, wherein the immunoassay is capable of detecting the presence of an ECGF antibody in a biological sample obtained from the subject having Lyme disease, and wherein the antibody is capable of binding to said antigen. The subject can be suffering from either antibiotic-refractory or antibiotic-responsive Lyme arthritis. The immunoassay may comprise an ELISA, agglutination test, direct immunofluorescence assay, indirect immunofluorescence assay, western blot, an immunoblot assay, and the like. The kit further comprises *Borrelia* antigens.

Also described herein is a kit for identifying a subject with chronic inflammatory arthritis as having Lyme arthritis, comprising ECGF or a set of synthesized ECGF peptides/epitopes and reagents necessary for conducting an immunoassay, wherein the immunoassay is capable of detecting the presence of T cell responses to said antigen in patients' biological samples. The subject can be suffering from either antibiotic-refractory or antibiotic-responsive Lyme arthritis. The immunoassay may comprise, but not limited to, ³H-thymidine incorporation assay, CFSE dilution, ELISpot, and the like.

The ECGF and ECGF peptides can include an ECGF peptide selected from the amino acid sequences LGRFERMLAAQGVDPG (SEQ ID NO: 1);
ADIRGFVAAVVNGSAQGAQI (SEQ ID NO:2); DKVSLVLAPALAACG (SEQ ID NO:3); SKKLVEGLSALVVDV (SEQ ID NO:4); KTLVGVGASLGLRVAAALTAMD (SEQ ID NO:5); LRDLVTTLGGALLWL (SEQ ID NO:6); and GTVELVRALPLALVLH (SEQ ID NO:7).

### DESCRIPTION OF THE DRAWINGS

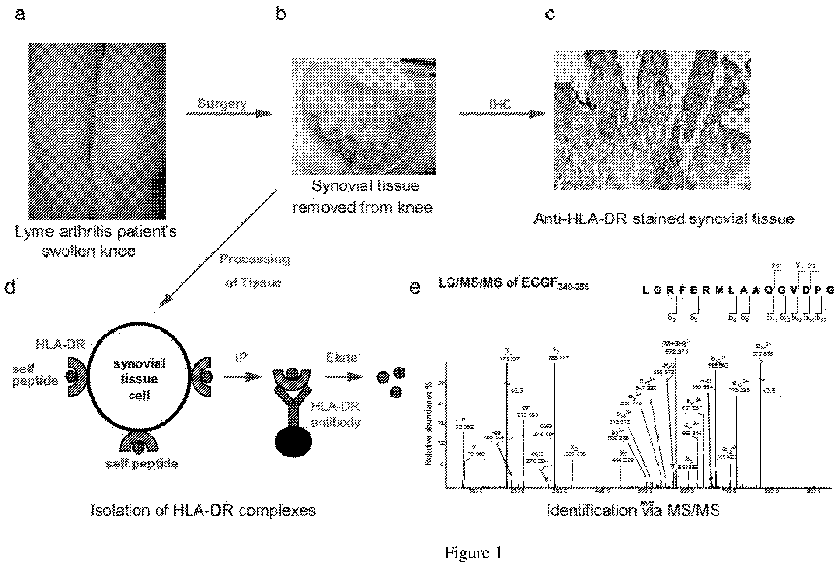

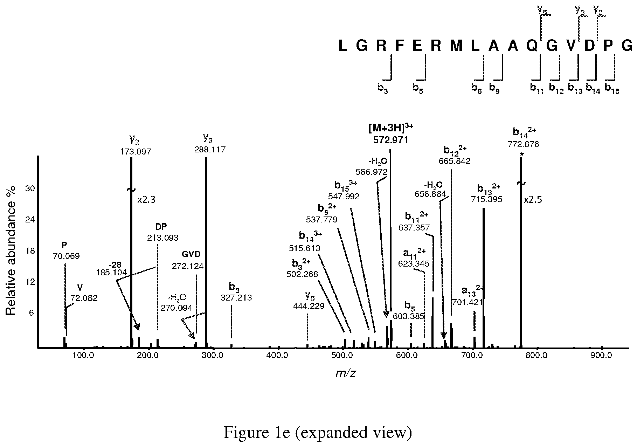

Figure 1 is an overview of the isolation and identification of *in vivo* HLA-DR presented peptides from patients' synovial tissue. Antibiotic-refractory Lyme arthritis usually manifests as one swollen knee (1a). In those cases in which joint swelling persists after ≥3 months of oral and intravenous antibiotics followed by a suboptimal response to disease-modifying antirheumatic drugs (DMARD), therapeutic arthroscopic synovectomies are sometimes performed. During the procedure, 20 g to 40 g of inflamed synovial tissue and subcutaneous fat are removed (1b). Immunohistologic staining of the synovial tissue shows marked exogenous expression of HLA-DR molecules (1c). The tissue is separated from the fat and HLA-DR complexes are immunoprecipitated from synovial cell lysates using a HLA-DR specific antibody (1d). HLA-DR presented peptides are eluted and identified by tandem mass spectrometry. In this case, the LC/MS/MS spectra of the ECGF₃₄₀₋₃₅₅ peptide are shown (1e).

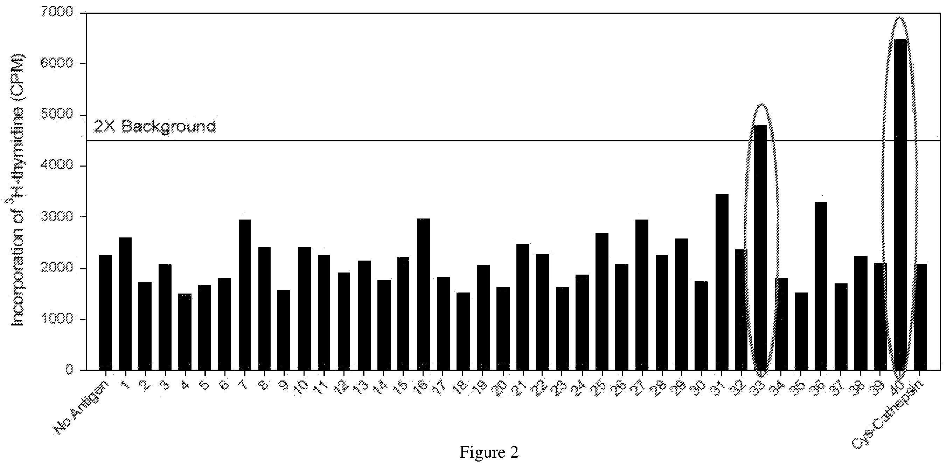

Figure 2 is a bar graph of data from the screening of 120 HLA-DR-presented peptides identified from the synovial tissue of one patient for T cell autoantigenicity using the patient's own T cells. PBMC obtained near the time of synovectomy were washed and resuspended in 200 µl of complete medium at a concentration of 2 x 10⁵ cells per well. All non-redundant HLA-DR presented peptides identified from the patient's synovial tissue sample were synthesized and tested in sets of three (2 µM of each peptide). After incubation for 5 days at 37°C and 5% CO₂, 0.5 µCurie of ³H-thymidine was added to each well, and cells were harvested 18 hours later to measure incorporation of ³H-thymidine into DNA. The data is presented as the average counts per minute (CPM) of duplicate wells for each peptide set (3 peptides/set, x-axis). A positive result was defined as a proliferative response > 2 times background.

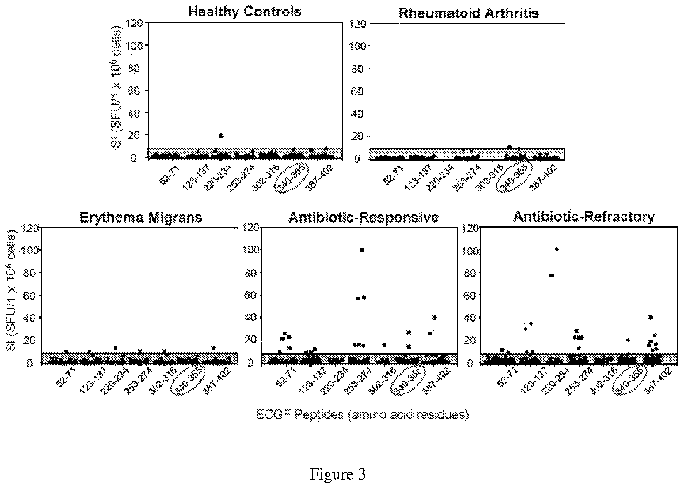

Figure 3 shows data testing PBMC from patients with antibiotic-refractory or antibiotic-responsive Lyme arthritis, RA or healthy control subjects for T-cell recognition of ECGF peptides. Cells of patients with Lyme arthritis were collected from patients seen over the past 12 years. PBMC were plated as described in Figure 2 and individual ECGF peptides (1 µM) were added to duplicate wells. After 5 days, cells were transferred to ELISpot plates previously coated with an IFN-γ capture antibody, and the assay was performed following the manufacturer's instructions (MabTech, OH, USA). Seven ECGF peptides, five of which were predicted to be promiscuous HLA-DR binders (binding more than 19 HLA-DR molecules, indicated in bold) were tested and the sequences were as follows:
   ⁵²**ADIRGFVAAVVNGSAQGAQI**⁷¹ (SEQ ID NO:2); **¹²³DKVSLVLAPALAACG**₁³⁷ (SEQ ID NO:3); ²²⁰SKKLVEGLSALVVDV²³⁴ (SEQ ID NO:4);
   ²⁵³**KTLVGVGASLGLRVAAALTAMD**²⁷⁴ (SEQ ID NO:5); ³⁴⁰**LGRFERMLAAQGVDPG**³⁵⁵ (SEQ ID NO:1) (this peptide, which is circled in red, was identified in LA1 synovial sample); ³⁰²LRDLVTTLGGALLWL³¹⁶ (SEQ ID NO:6); and ³⁸⁷**GTVELVRALPLALVLH**⁴⁰¹ (SEQ ID NO:7). The five peptides predicted to be promiscuous binders were tested in all patients or control subjects, whereas the 2 non-promiscuous binders were tested in only a subset of patient or control cells (i.e., 15 of 18 healthy control subjects, none of the 12 RA patients, 18 of 19 patients with EM, 7 of 28 antibiotic-responsive arthritis patients, and 12 of 38 antibiotic-refractory arthritis patients) due to limited availability of cells.

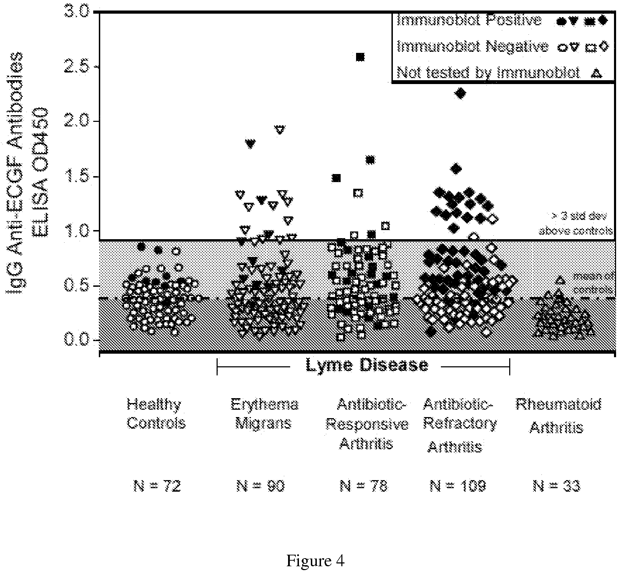

Figure 4 shows IgG anti-ECGF autoantibody responses in the sera of patients with various manifestation of Lyme disease, RA or healthy control subjects. Cells of patients with Lyme disease were collected from patients seen over the past 25 years. IgG anti-ECGF autoantibodies in serum samples were determined by both ELISA and immunoblotting. *See* Examples, below for details.

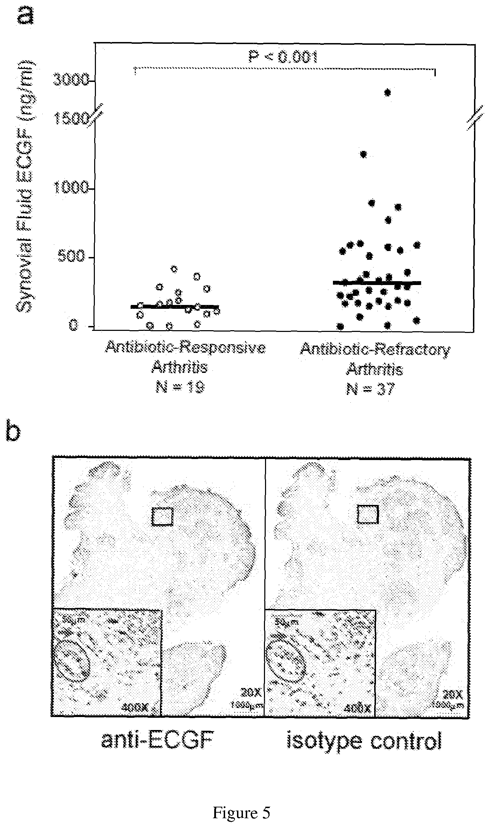

Figure 5 shows detection of ECGF protein in the synovial fluid of antibiotic-responsive, antibiotic-refractory and the synovial tissue of an antibiotic-refractory Lyme arthritis patient. (5a) reflects ECGF concentrations in synovial fluid measured by sandwich ELISA. (5b) Representative serial synovial tissue sections from an antibiotic-refractory Lyme arthritis patient stained with anti-ECGF or isotype control antibodies. Immunohistochemical staining of ECGF was moderate to intense in both the synovial lining and sublining of antibiotic-refractory Lyme arthritis patients (20X). At a higher magnification (400X), positive ECGF staining can be observed in the cytoplasm and nucleus of fibroblast-like cells in the lining (arrow). Intense staining of ECGF was seen in areas surrounding microvessels (circled) in synoival sublining.

### DETAILED DESCRIPTION

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

As used herein and in the claims, the singular forms include the plural reference and vice versa unless the context clearly indicates otherwise. Other than in the operating examples, or where otherwise indicated, all numbers expressing quantities of ingredients or reaction conditions used herein should be understood as modified in all instances by the term "about."

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

Antibiotic-refractory Lyme arthritis, defined as persistent synovitis for months to years after spirochetal killing with antibiotic therapy, is hypothesized to result from B. *burgdorferi-*induced autoimmunity. Lyme borreliosis, the most common tick-borne disease in the northern hemisphere, results from infection with spirochetes of the *Borrelia burgdorferi sensu lato* complex. In the U.S., *Borrelia burgdorferi sensu stricto* (*Bb*) is the sole cause of the illness, whereas in Europe, *Borrelia afzelii* (*Ba*) and *Borrelia garinii* (*Bg*) are the primary pathogens. Steere, 345 N. Engl. J. Med. 115 (2001); Baranton et al., 42 Intl. J. Syst. Bacteriol. 378 (1992); Canica et al., 25 Scand. J. Infect. Dis. 441 (1993).

Infection with each of these species usually begins with a slowly expanding skin lesion, called erythema migrans (EM), which occurs at the site of the tick bite. In the U.S., where *Bb* is particularly arthritogenic, about 60% of un-treated patients develop intermittent or chronic joint swelling in later months, especially affecting knees. Steere et al., 107 Annals Internal Med. 725 (1987). These patients can usually be treated successfully with 1 month of oral or intravenous (IV) antibiotics, an outcome called antibiotic-responsive arthritis. Steere et al., 37 Arthritis Rheum. 878 (1994); Dattwyler et al., 117 Wiener klinische Wochenschrift 393 (2005). In a small percentage of patients, however, proliferative synovitis persists for months or several years after apparent spirochetal killing with >2 months of oral antibiotics, >1 month of intravenous antibiotics, or usually both, referred to as antibiotic-refractory arthritis. Steere & Angelis, 54 Arthritis Rheum. 3079 (2006a). This disease course is hypothesized to result from infection-induced autoimmunity.

Evidence supporting the hypothesis that Lyme arthritis is an infection-induced autoimmunity disease is 3-fold: (1) PCR and culture results of synovectomy specimens have been uniformly negative (Nocton et al., 330 New. Engl. J. Med.229 (1994)); (2) relapse of infection has not been observed with the use of DMARDs after antibiotic therapy (Steere & Angelis, 2006a); and (3) specific HLA-DR alleles are the greatest known genetic risk factor for antibiotic-refractory arthritis⁹, a risk factor commonly associated with autoimmune diseases. Steere et al., 203 J. Experi. Med. 961 (2006b).

A number of spirochetal and host risk factors associated with antibiotic-refractory Lyme arthritis. These factors include infection with highly inflammatory *B. burgdorferi* RST1 (OspC type A) strains; a host Toll-like receptor 1 (TLR1) polymorphism (1805GG) which leads to exceptionally high inflammatory responses; certain HLA-DR molecules, such as the DRB 1*0401 molecule, that bind an epitope of *B. burgdorferi* outer-surface protein A (OspA₁₆₅₋₁₇₃); high levels of inflammatory cytokines and chemokines in joint fluid, particularly CXCL9 and CXCL10, which are chemoattractants for T_{H}1 effector cells; and a dominant T_{H}1 response in joint fluid with persistently low percentages of Treg. The present invention adds to these factors a biomarker, the antigen ECGF, which is present and induces autoreactive immune cells in many, but not all, antibiotic-refractory Lyme arthritis patients.

As with all forms of chronic inflammatory arthritis, including RA, the synovial lesion in in patients with antibiotic-refractory arthritis shows synovial hypertrophy, vascular proliferation, infiltration of mononuclear cells, and intense expression of human leukocyte antigen D-related (HLA-DR) molecules. In antibiotic-refractory Lyme arthritis, the HLA-DR risk alleles include HLA-DRB1*0101, 0401, 0404, 0405 and 1501/dry5*0101 (Steere et al., 2006b), similar to those in rheumatoid arthritis (RA) (Deighton et al., 36 Clin. Genet. 178 (1989); Seldin et al., 42 Arthritis Rheum. 1071 (1999)). HLA-DR molecules present peptides, both foreign and self, to CD4+ T-cells, which leads to T-cell activation and proliferation. With tissue-specific autoimmune diseases, HLA-DR molecules in the target tissue, in this case synovial tissue, are thought to present disease-related autoantigenic epitopes, but for the most part these epitopes have not yet been identified.

Initial attempts to uncover autoantigens in antibiotic-refractory Lyme arthritis were based upon a search for molecular mimicry between an epitope of *Bb* outer-surface protein A (OspA₁₆₃₋₁₇₅), which is bound by refractory arthritis-associated HLA-DR molecules, and those in human proteins. Steere et al., 52 Clin. Inf. Dis. S259 (2011). The first candidate autoantigen identified was LFA-1α_{L332-340} (Gross et al., 281 Sci. 703 (1998)), which has sequence homology with six of the nine core amino acid residues in OspA₁₆₃₋₁₇₅; and later, MAWD-BP₂₈₀₋₂₈₈ which was shown to have sequence identity with eight of the nine core OspA residues. Drouin et al., 45 Molec. Immunol. 108 (2008). Only a minority of patients had T-cell reactivity with these peptides, however, and none had B-cell responses to these self proteins. *Id.;* Steere et al., 48 Arthritis Rheum. 534 (2003). Others, using recombinant antibody probes derived from B-cells in patients' synovial lesions, identified cytokeratin 10 as a candidate autoantigen. Ghosh et al., 117 J. Immunol. 2486 (2006). This probe cross-reacted with OspA, but only three of fifteen patients with antibiotic-refractory arthritis had slight antibody responses to this self protein. Hence, none of these known candidate autoantigens appeared to explain antibiotic-refractory Lyme arthritis.

The identification of *in vivo* HLA-DR-presented peptides in synovial tissue was developed as a novel, unbiased approach that combines discovery-based proteomics with translational research for the identification of immunogenic self antigens. This protocol has steps comprising: (a) a proteomics approach utilizing tandem mass spectrometry (MS/MS) for the identification of HLA-DR-presented peptides in individual subject's synovial tissue; (b) synthesis and testing of all peptides identified in step (a) for reactivity with the same subject's PBMC, thereby asking the subject's own T-cells to indicate which HLA-DR self-peptides may be acting as autoantigens; and (c) validation that any immunogenic peptides and their source proteins identified in a single subject in step B also induce T- and B-cell reactivity in large numbers of subjects with Lyme arthritis.

The initial results based on mass spectrometry analysis of HLA-DR-eluted peptides from synovial tissue from four patients, two with antibiotic-refractory arthritis and two with RA were reported in Seward et al., 10 Molec. Cell Proteomics M110 002477 (2011). This approach identified 1,427 synovial HLA-DR *in vivo*-presented peptides (220 to 464 per patient), which on average represents a ten-fold increase in peptide identification compared with four previous studies of other tissues or fluids. *See* Gordon et al., 25 Eur. J. Immunol. 1473 (1995); Muixi et al., 181 J. Immunol. 785 (2008); Oshitani et al., Intl. J. Molec. Med. 99 (2003); Wahlstrom et al., 117 J. Clin. Investig. 3576 (2007). The 1,427 peptides were derived from 166 source proteins, including a wide range of intracellular and plasma proteins. These source proteins were substantially different than those identified previously from EBV cell lines.

All non-redundant HLA-DR-presented peptides identified from individuals were synthesized for testing with the same subjects' PBMC. The first subject peptides tested came from a youth with antibiotic-refractory Lyme arthritis (LA1) who had a synovectomy following persistent arthritis for one year, despite 9 months of oral and intravenous antibiotic therapy. He had one of the refractory arthritis-associate alleles: DRB1*0101. Of the 2,237 MS/MS spectra generated from his tissue sample, 464 had a consensus match identified with two or more mass spectrometry search programs (Mascot, OMSSA or X! Tandem), of which 104 were non-redundant. These 104 peptides, along with sixteen additional peptides identified by only one of three search programs, were synthesized and tested in a T-cell proliferation assay using the patient's own PBMC. Because of limited cell numbers, peptides were pooled (three per well) for testing.

Only two peptide sets (set 33 and set 40) induced proliferation responses that were >2 times background (Figure 2). Enough cells remained for retesting the first set together and the second peptide set individually. In the second assay, only one peptide induced proliferation and IFN-γ secretion that were >2 times background. This peptide
(³⁴⁰LGRFERMLAAQGGVDPG³⁵⁵) (SEQ ID NO:1), 1 of the 16 identified by only one of the three search programs, originated from the source protein platelet-derived endothelial cell growth factor (ECGF), also called thymidine phosphorylase or gliostatin.

*In vitro*, ECGF acts as a chemotactic factor, causes a proliferative effect on endothelial cells, (Ishikawa et al., 338 Nature 557 (1989)); inhibits the growth of glial cells; and contributes to cortical neuron survival. Asai et al., 267 J. Biological Chem. 20311 (1992). *In vivo,* ECGF induces angiogenesis (Ishikawa et al., 1989); and is often over-expressed in many human cancers. Bronckaers et al., 29 Med. Res. Rev. 903 (2009). ECGF enzymatic activity involves the conversion of thymidine to thymine and deoxyribose-1-phosphate, and the thymidine-derived sugar is postulated to contribute to angiogenesis. Bijnsdorp et al., Biochemical Pharmacol. 786 (2010). Interestingly, most previously identified autoantigens in endocrine autoimmune diseases also have enzymatic activity. Aletha et al., 62 Arthritis Rheum 2569 (2010). ECGF, however, has not been identified previously as an autoantigen in any disease.

PBMC and serum samples from large numbers of patients with various manifestations of Lyme disease, from a rheumatoid arthritis comparison group, and from healthy control subjects, were tested for T- and B-cell reactivity with ECGF. All Lyme disease patients met the criteria of the Centers for Disease Control and Prevention for the diagnosis of this infection (39(RR-13) MMWR Morb.Mortl.Wkly Rpts. 1 (1990)), and those with RA met the ACR/EULAR criteria. Aletha et al., 2010. *Bb* was isolated from the skin lesions of all patients with EM tested here, and all patients with Lyme arthritis had high antibody responses to many *Bb* proteins by ELISA and immunoblotting. All control subjects were seronegative for *Bb* infection.

Initially, subjects' PBMC were tested using commercially available recombinant ECGF. ECGF inhibited thymidine incorporation into actively dividing cells (Takeuchi et al., 37 Arthritis Rheum. 662 (1994)), but it non-specifically induced PBMC to secrete IFN-γ. Consequently, three HLA-DR T-cell epitope prediction algorithms (Wang et al., PLoS Computat. Biol. e1000048 (2008)), were used to identify seven ECGF peptides, including the one peptide identified in the initial patients' sample (ECGF₃₄₀₋₃₆₅), that were predicted to be presented by the HLA-DR molecules associated with antibiotic-refractory Lyme arthritis. These peptides were synthesized and tested for autoreactivity via IFN-γ ELISPOT assays using patient and control PBMC (Figure 3). Based upon the values obtained in 14 healthy control subjects (Figure 3), a positive T cell response was defined as a stimulation index (no. of spots induced by an ECGF peptide /no. of spots in no antigen controls) 3 standard deviation (SD) above the mean of healthy controls, which characteristically gave >40 spot forming units (SFU)/10⁶ PBMC and a stimulation index of >8.

Only one of eighteen healthy control subjects (6%) had a low-response to a single ECGF peptide. In comparison, three of nineteen subjects with EM (16%) had responses which were slightly above background (P=0.6), eight of twenty-eight subjects with antibiotic-responsive arthritis (29%) had higher responses to ECGF peptides (P = 0.07), and fourteen of thirty-eight subjects with antibiotic-refractory arthritis (36%) had similarly high responses to these peptides (P = 0.02). Moreover, ten subjects with antibiotic-responsive or antibiotic-refractory arthritis had reactivity with two to four ECGF peptides, suggestive of epitope spreading. In comparison, only two of twelve patients with RA (17%) had reactivity with a single epitope that was only slightly above background. These results suggested that T cell autoreactivity to ECGF may be specific for Lyme disease; it may begin early in the infection, and it increases in frequency, magnitude and number of epitopes recognized later in the illness in patients with Lyme arthritis. Moreover, because only ∼20% of the ECGF protein sequence was tested in this analysis, the actual percentage of Lyme arthritis patients with ECGF T cell autoreactivity may be higher.

For an autoantigen to contribute to pathogenicity, it likely would need to induce both T- and B-cell responses. Therefore, IgG anti-ECGF antibodies were in patients' serum samples using two methods, ELISA and immunoblotting. A positive response by ELISA was defined as >3 SD above the mean value of healthy control subjects, and a positive immunoblot was defined by the presence of a band at the correct location for ECGF.

As determined by immunoblotting, serum samples from nine of seventy-two healthy control subjects (13%) had weak reactivity with ECGF (Figure 4). In contrast, fifty of 109 patients (46%) with antibiotic-refractory arthritis had weak-to-strong responses to ECGF, which was significantly different than healthy subjects (P<0.001); twenty of seventy-eight patients (26%) with antibiotic-responsive arthritis and 10 of 90 patients (11%) with EM also had ECGF responses, but these frequencies were not significantly different than control subjects (P=0.07 and 1.0, respectively).

As determined by ELISA, none of seventy-two healthy control subject had a positive response compared with eighteen of 109 patients (17%) with refractory arthritis (P<0.001), seven of seventy-eight patients (9%) with responsive arthritis (P=0.03), fifteen of ninety EM patients (16%) (P<0.001), and none of thirty-three RA patients. Moreover, most of the patients in the refractory group who had an ELISA value above the mean value had a positive immunoblot, which was not the case in the other groups. Thus, autoantibodies to ECGF were sometimes present early in the infection or later in the illness in patients with antibiotic-responsive arthritis, but the frequency of these antibodies was significantly greater than healthy controls only in patients with antibiotic-refractory arthritis

For the autoantigen ECGF to have pathogenic relevance in antibiotic-refractory Lyme arthritis, one would predict that this protein would be present in high concentrations in patients' inflamed joints. Therefore, ECGF levels in joint fluid were determined by ELISA and its presence in synovial tissue identified using immunohistochemical techniques. Although joint fluid was available in patients with antibiotic-responsive arthritis, synovial tissue was not as therapeutic synovectomies are never necessary in this patient group.

As determined by sandwich ELISA, subjects with antibiotic-refractory arthritis had very high concentrations of ECGF in synovial fluid (mean value = 328 ng/ml ± 428), and these concentrations were significantly higher than those in subjects with antibiotic-responsive arthritis (142 ng/ml ± 119) (P<0.001) (Figure 5a). Previously, other investigators showed that ECGF concentrations were also high in the joint fluids of RA patients (223 ng/ml±211, N=248), (Takeuchi et al., 37 Arthritis Rheum. 662 (1994)), similar to those levels found in patients with antibiotic-refractory Lyme arthritis. In contrast, osteoarthritis (OA) patients, a minimally inflammatory form of arthritis, had significantly lower ECGF levels (8.7 ng/ml ± 14.3) than subjects with either antibiotic-refractory arthritis or rheumatoid arthritis.

Synovial tissue samples from sixteen patients with antibiotic-refractory arthritis and five with RA were analyzed for the presence of ECGF. Of the sixteen patients with antibiotic-refractory arthritis, ten (63%) had moderate-to-intense staining for ECGF in the lining and sublining of the synovial tissue, four (25%) had mild staining, and two (12%) had no staining in these areas. A representative example of intense staining at these sites is shown in Figure 5b. At high magnification (400X), ECGF staining was clearly evident in the sublining area around blood vessels (circle) and in large cells that were likely synovial fibroblasts (arrow). In contrast, three of the five patients with RA had only mild staining in lining areas, and two had only mild staining around blood vessels. Thus, in the majority of patients with antibiotic-refractory arthritis, large amounts of ECGF were present in joint fluid and synovial tissue where it could be presented by HLA-DR molecules on local antigen presenting cells.

The duration of arthritis prior to and after antibiotic therapy, HLA-DR alleles, and joint fluid cytokine and chemokine levels were compared in patients with antibiotic-refractory or antibiotic-responsive arthritis according to ECGF antibody responses, as shown in Table 1:

**Table 1. Clinical findings: antibiotic-refractory or -responsive Lyme arthritis**

| | Antibiotic-refractory | Antibiotic-responsive | P value |
|---|---|---|---|
| Total no. of patients | 109 | 78 | |

| **Duration of arthritis** | | | |
|---|---|---|---|
| (mos.), median (range) | | | |
| Onset to antibiotics | | | |
| Antibiotics to resolution | 11(4-48) | 2(1-3) | <0.001 |

| **Antibody responses** | | | |
|---|---|---|---|
| according to HLA-DR alleles | | | |
| No. of patients tested | 97 | 70 | |
| No. of alleles (%) | 194 | 140 | |
| 0101,0102,04* or 1501 | 99(51%) | 53(38%) | 0.02 |
| 0301,0801,1101, or 1104 | 36(19%) | 42(30%) | 0.02 |
| 0701 | 24(12%) | 13(9%) | 0.5 |

| **Cytokines/chemokines**** | | | |
|---|---|---|---|
| No. of patients tested | 37 | 19 | |
| Levels in joint fluid, pg/ml | | | |
| IFNγ | 13 | 3 | 0.01 |
| TNFα | 28 | 18 | 0.05 |
| IL-1β | 4 | 2 | 0.05 |
| IL-6 | 20,900 | 6,920 | 0.02 |
| IL-8 | 13,800 | 4,200 | 0.01 |
| CXCL9 | 354,000 | 119,200 | 0.03 |
| CXCL10 | 43,700 | 23,450 | <0.001 |
| CCL2 | 3,570 | 1,085 | <0.001 |
| CCL3 | 258 | 78 | 0.006 |
| CCL4 | 307 | 144 | 0.007 |

| | | | |
|---|---|---|---|
| *The 04 alleles in this analysis were 0401, 0402, 0403, 0404, 0405, 0407, 0408, and 0409. ** Levels of the following cytokines and chemokines were also determined: CCL5, CXCL11, CXCL13, IL-7, IL-10, IL-12p40, IL-12p70, IL-17, IFNα, but the differences between the values in antibiotic refractory and responsive patients were not statistically significant. | | | |

Consistent with previous studies (Steer & Angelis, 54 Arthritis Rheum. 3079 (2006); Steere et al., 203 J. Experi. Med. 961 (2006); Shin et al., 56 Arthritis Rheum. 1325 (2007)), a number of significant differences were noted in these factors between patients with refractory or responsive arthritis but within each of these groups, significant differences were not found according to ECGF antibody responses. There was a trend, however, toward higher levels of CXCL9 in the sixteen patients who had positive ECGF antibody responses (determined by immunoblotting) compared with the twenty-four patients who lacked such responses (median value, 636,500 vs 313,000 pg/ml).

When the levels of 20 cytokines and chemokines in joint fluid were correlated with the joint fluid ECGF levels, a very strong direct correlation between the levels of IFN-γ and ECGF were found in patients with antibiotic-refractory arthritis (r = 0.8, P=0.0000000001), as shown in Table 2:

**Table 2. Correlation of ECGF protein levels with cytokines and chemokines concentrations in joint fluid in patients with antibiotic-refractory and antibiotic-responsive Lyme arthritis**

| | Antibiotic-refractory* | | | Antibiotic-responsive | | |
|---|---|---|---|---|---|---|
| | No. samples w/ detectable cytokine levels | Correlation Coefficient < value | P value | No. samples w/ detectable cytokine levels | Correlation Coefficient < value | P value |
| Pro-inflammatory cytokines | | | | | | |
| IFN-γ | 37 | 0.8 | 0.00000000001 | 18 | 0.1 | 0.6 |
| IFN-α | 26 | 0.6 | 0.003 | 15 | -0.07 | 0.8 |
| TNF | 36 | 0.7 | 0.00001 | 19 | 0.2 | 0.4 |
| IL-1β | 37 | 0.6 | 0.0002 | 18 | 0.03 | 0.9 |
| | | | | | | |
| Macrophage chemoattractants | | | | | | |
| CCL3 | 36 | 0.6 | 0.0002 | 16 | 0.09 | 0.7 |
| CCL4 | 36 | 0.5 | 0.0008 | 19 | 0.2 | 0.4 |
| | | | | | | |
| Anti-inflammatory cytokines | | | | | | |
| IL-10 | 36 | 0.5 | 0.0007 | 18 | 0.2 | 0.5 |
| IL-5 | 36 | 0.9 | 0.00000000004 | 12 | -0.1 | 0.7 |

| | | | | | | |
|---|---|---|---|---|---|---|
| *Only cytokines and chemokines are shown that correlated significantly with ECGF protein levels. Correlations were not found between ECGF values and the levels of CXCL9, CXCL10, CXCL11, CXCL13, IL-6, IL-7, IL-8, IL-12p40, IL-12p70, IL-17, IL-23, CCL2, and CCL5. Total number of patients' joint fluid samples tested were 37 antibiotic-refractory and 19 antibiotic-responsive. | | | | | | |

Similarly, there was a strong direct correlation in the refractory group between IL-5 and ECGF levels. In another system, a known autoantigen (thyroglobulin) induced patients' PBMC to secrete both IFNγ and IL-5, and IL-5 may enhance autoantibody production. Nielsen et al., 147 Clin. Exp. Immunol. 287 (2007). There was also a direct correlation in the refractory group between the levels of ECGF and several other inflammatory or anti-inflammatory cytokines and chemokines, but these correlations were not as strong as those with IFN-γ and IL-5. In contrast, there was no correlation between the levels of these inflammatory mediators and ECGF values in patients with antibiotic-responsive arthritis. The strong correlation between the levels of IFN-γ and ECGF in joint fluid suggests that IFN-γ might also have a role in inducing secretion of the autoantigen ECGF in the synovial lesion.

The present invention provides for a novel biomarker, ECGF, that is present in high concentrations in inflamed joints and induces T- and B-cell responses in some subjects with Lyme arthritis, particularly in those with antibiotic-refractory arthritis. ECGF might be considered a surprising autoantigen because its expression is not joint-specific. The K/BxN mouse model of autoimmune arthritis may also hold insights regarding this issue. In that model, joint-specific inflammation is due to immune recognition of the ubiquitous self-protein, glucose-6-isomerase (GPI), which is recognized by the single T-cell receptor in this transgenic mouse. Matsumoto et al., 3 Nat. Immunol. 360 (2002). GPI, which accumulates in high concentrations on articular surfaces, is bound by anti-GPI autoantibodies, which triggers the activation of complement and Fc receptors leading to autoimmune synovitis. Individual anti-GPI monoclonal antibodies do not induce arthritis, however; rather, anti-GPI antibodies recognizing multiple epitopes are required. Maccioni et al., 195 J. Exp. Med. 1971 (2002). Thus, although GPI is a ubiquitous self-antigen, pathology develops only in the localized environment of the joint where inflammatory responses to this protein are not regulated appropriately.

In comparison, human autoimmune disease is more complicated due to the diversity of immune responses. In RA, anti-cyclic citrullinated peptide (anti-CCP) antibodies, the first autoantibodies identified that are specific for this disease, are found in about 60% of RA patients (Lee & Schur, 62 Annals Rheum. Disease 870 (2003)), suggesting that other as yet to be identified autoantibodies may play a role in the disease. Moreover, anti-CCP antibodies may develop months or years before the onset of arthritis (Nielen et al., 50 Arthritis Rheum. 380 (2004)), suggesting that these antibodies may be necessary but are not sufficient to induce arthritis. Similarly, in antibiotic-refractory Lyme arthritis, antibody responses to ECGF were found significantly more often in patients with antibiotic-refractory arthritis, but not all refractory patients had anti-ECGF antibody responses. Furthermore, ECGF reactivity was sometimes found in patients with other early or late manifestations of the illness, but these patients did not have clinical autoimmune disease. Thus, as in RA, pathogenicity in antibiotic-refractory Lyme arthritis surely involves heterogenous, multifactorial processes; and other yet to be identified factors, including other specific autoantigens, may play a role in the pathogenesis of the disease.

There are a number of risk factors involved in antibiotic-refractory Lyme arthritis, including both spirochetal and host factors. For example, this disease course is triggered significantly more often by *Bb* RST1 strains, which account for 30%-50% of the infections in the northeastern U.S. Jones et al., 60 Arthritis Rheum. 2174 (2009). These strains are more inflammatory than other strains, including those found in Europe (Strle et al., 200 J. Infect. Dis. 1936 (2009)), particularly in individuals with a TLR1 polymorphism. Strle et al., Abstract 12th Intl. Cong. Lyme Borreliosis Tick-Borne Dis. 12 (2010). Further, RST1-infected, antibiotic-refractory patients with this polymorphism had exceptionally high levels of IFNγ and the IFNγ-inducible chemoattractant CXCL9 in joint fluid. Shine et al., 56 Arthritis Rheum. 1325 (2007). Also, *Bb,* an extracellular pathogen, is known to bind certain host proteins (Hallstrom et al., 202 J. Infect. Dis. 490 (2010)), or tick proteins (Anguita et al., 19 Immun. 849 (2002)), to its surface to aid in its spread and survival. Perhaps ECGF binds directly to the surface of certain spirochetal strains resulting in simultaneous uptake and processing by antigen-presenting cells.

Alternately, T-cell epitope mimicry between a spirochetal and ECGF epitope presented by certain HLA-DR alleles might account for ECGF autoreactivity, although this seems less likely since no single ECGF epitope was recognized by all or even the majority of ECGF-reactive patients. Studies in an animal model of antibiotic-refractory Lyme arthritis suggest that certain HLA-DR alleles, such as DRB 1*0401, lead to greater inflammatory responses. Iliopoulou et al., 60 Arthritis Rheum. 3831 (2009). Once established, the role of immune reactivity with ECGF may be to amplify joint inflammation, particularly since large amounts of this antigen are present in inflamed joints. Finally, an antibiotic-refractory course surely requires dysregulation of immune responses. It was shown previously that Th1 cells are abundant and enriched in synovial fluid in both antibiotic-refractory and antibiotic-responsive patients. In the refractory group, lower numbers of Treg correlated with slower resolution of arthritis. Shen et al., 62 Arthritis Rheum. 2127 (2010). High levels of IFNγ in joints, secretion of ECGF, upregulation of HLA-DR molecules, and high concentrations of the CD4+ T effector cell chemoattractant CXCL9 may help set the stage for the development of autoimmunity to ECGF.

The identification of ECGF for a biomarker in Lyme arthritis, as provided herein, is an important addition to the clinician's arsenal in combating chronic inflammatory arthritis, and assists the clinician in choosing the course of therapy. For example, when antibiotic-refractory Lyme arthritis is diagnosed, nonsteroidal anti-nflammatory drugs (NSAIDs) or intra-articular steroid; or disease modifying anti-rheumatic drugs (DMARDs), such as hydroxychloroquine or methotrexate, may be prescribed. Additionally or alternatively, anti-TNF therapy (e.g., HUMIRA® (adalimumab) or ENBREL® (etanercept)) may be beneficial. It is also possible that ECGF-specific therapy, such as targeting ECGF and/or ECGF-binding antibodies, may also be beneficial.

Importantly, the discovery-based methodology used herein to identify novel autoantigens is likely to have much broader applicability. The present invention shows that HLA-DR-presented peptides may be identified from synovectomy specimens of individual patients by tandem mass spectrometry, and immunogenic antigens may be uncovered in these patients by synthesis and testing of these peptides with the same patient's PBMC. This approach can be applicable to any form of chronic inflammatory arthritis, any autoimmune disease, or malignancy in which important immune responses are not yet known.

The present embodiments provide for a biomarker useful for the diagnosis of Lyme arthritis, in particular, antibiotic-refractory Lyme arthritis. More specifically, subject immunoreactivity (e.g., ECGF-reactive T-cells, and/or anti-ECGF antibodies) to ECGF or ECGF peptides, such as, for example, LGRFERMLAAQGVDPG (SEQ ID NO: 1), are indicative of Lyme arthritis in some, but not all, Lyme arthritis subjects. Additional ECGF peptides useful according to the present embodiments include, for example, ADIRGFVAAVVNGSAQGAQI (SEQ ID NO:2); DKVSLVLAPALAACG (SEQ ID NO:3); SKKLVEGLSALVVDV (SEQ ID NO:4); KTLVGVGASLGLRVAAALTAMD (SEQ ID NO:5); LRDLVTTLGGALLWL (SEQ ID NO:6); and GTVELVRALPLALVLH (SEQ ID NO:7).

For example, an immunoassay can be used to identify antibodies present in a serum sample that bind ECGF. The immunoassay can be an ELISA, agglutination test, direct immunofluorescence assay, indirect immunofluorescence assay, western blot, an immunoblot assay, and the like. For example, the immunoassay could be an immunoblot that carries a recombinant ECGF or ECGF peptide(s). The immunoassay can also include or be used with an immunoassay (e.g., a kit) that include *Borrelia* antigens known in the art, such as p83/100 derived from strain PKo (*Borrelia afzelii*); p39 (BmpA) and OspC from strains PKa2 *(B. burgdorferi sensu stricto*), PBi (*B*. *garinii,* OspA-type 4), and PKo; p41i (internal flagellin fragment) from PKo and PBi; p58 derived from PBi; Osp17 from PKo; decorin binding protein A (DbpA) derived from *B. garinii* strain PBr (OspA-type 3); VlsE from *B. burgdorferi sensu stricto* strain PKa2; and/or OspC from *B. garinii* strain 20047. *See, e.g.,* 41 J. Clin. Microbiol. 1299 (2003); Wilske et al., 188 Med. Micriobiol. Immunol. 139 (1999). It should be noted that the *Borrelia* antigen(s) can be derived from natural and/or recombinant sources. For example, the present ECGF antigen can be included in or used in conjunction with an immunoassay such as the BORRELIA VIRASTRIPE® IgG, IgM test kit (Viramed Biotech AG, Planegg, Germany). The BORRELIA VIRASTRIPE® is an immunoblot that carries native, purified antigens from *Borrelia afzelii* (Pko), *Borrelia burgdorferi sensu stricto,* and recombinant *Borrelia* antigen VlsE. Alternatively, the present ECGF antigen may be included in or used in conjunction with an immunoassay such as the BORRELIA B31 VIRABLOT® Western blot test kits (Viramed Biotech AG, Planegg, Germany) which identify anti*-Borrelia* antigen-binding IgG and/or IgM in the serum of suspected *Borrelia-*infected patients. For immunoassays designed to identify ECGF antigen-binding antibodies in the serum of inflammatory arthritis subjects, the serum sample can be pre-enriched for antibodies by methods known in the art.

Further, ECGF-reactive T-cells in PBMC and SFMC can be assessed using a number of assays. For example, ECGF-reactive T-cells in PBMC and SFMC can be assessed using tetramer reagents comprising recombinant HLA-DR molecules and ECGF epitopes. "Epitope" refers to that portion of any molecule capable of being recognized by, and bound by, an antibody (the corresponding antibody binding region may be referred to as a paratope), and/or eliciting an immune response. In general, epitopes consist of chemically active surface groupings of molecules, e.g., amino acids, and have specific three-dimensional structural characteristics as well as specific charge characteristics.

The gene for human ECGF has been sequenced and is available at numerous sources such as the NCBI web site, GeneID: 1890 (UniProtKB/Swiss-Prot: P19971). Further, the ECGF gene is conserved in human, chimpanzee, rat, and zebrafish. Hence, ECGF peptides can include those derived from non-human sources or appropriate sequence information. In an aspect of the invention, the ECGF antigen is predicted to be presented by HLA-DR molecules associated with chronic inflammatory arthritis.

It is possible to determine the three dimensional structures of proteins of up to about 15 kDa by nuclear magnetic resonance (NMR). The technique only requires a concentrated solution of pure protein. No crystals or isomorphous derivatives are needed. The structures of a number of proteins have been determined by this method. The details of NMR structure determination are well-known in the art. *See, e.g.,* Wuthrich, NMR of Proteins & Nucleic Acids (Wiley, N.Y., 1986); Wuthrich, 243 Science 45 (1989); Clore et al., 24 Crit. Rev. Bioch. Molec. Biol. 479 (1989); Cooke et al., 8 Bioassays 52 (1988).

One embodiment of the present invention provides for a kit for identifying a subject with Lyme arthritis, comprising ECGF or a set of synthesized ECGF peptides, and reagents necessary for conducting an immunoassay, wherein the immunoassay is capable of detecting the presence of an antibody in a sample obtained from said subject that binds to ECGF or the set of synthesized ECGF peptides.

ECGF is available commercially, for example from R&D Systems, Inc. (Minneapolis, MN). Additionally, ECGF can be obtained from platelets, liver, lung, placenta, spleen, lymph nodes, peripheral lymphocytes, and astrocytes. *See* Haraguchi et al., 368 Nature 198 (1994); Toi et al., 6 Lancet Oncol. 158 (2005); Akiyama et al., 95 Canc. Sci. 851 (2004).

In a particular embodiment, the ECGF peptides are one or more of the peptides having the following amino acids: LGRFERMLAAQGVDPG (SEQ ID NO: 1); ADIRGFVAAVVNGSAQGAQI (SEQ ID NO:2); DKVSLVLAPALAACG (SEQ ID NO:3); SKKLVEGLSALVVDV (SEQ ID NO:4); KTLVGVGASLGLRVAAALTAMD (SEQ ID NO:5); LRDLVTTLGGALLWL (SEQ ID NO:6); GTVELVRALPLALVLH (SEQ ID NO:7). The immunoassay in the kit may be an enzyme-linked immunosorbent assay (ELISA) or immunoblot, the components for which are well-known in the art. The sample obtained from the subject may be peripheral blood, serum, synovial fluid, synovial tissue, peripheral blood mononuclear cells (PBMC), or the synovial fluid mononuclear cells (SFMC). The subject may be a mammal, such as a human. The peptides may be synthesized or obtained from natural or recombinant sources, each of which is well-known in the art.

Also described herein is a method for determining whether a biological sample, (for example, a sample comprising PBMC or SFMC), bears T-cells reactive to ECGF or ECGF peptides or epitopes by providing a set of at least one synthesized ECGF peptide or epitope that are predicted to be presented by HLA-DR molecules; stimulating the biological sample with at least one of the ECGF peptides/epitopes (or its functionally equivalent portion, analog, or derivative); and measuring T-cell proliferation *in vitro* or secretion of IFN-γ into cell culture supernatants as a test for T-cell reactivity.

Another embodiment provides for a method for determining whether a subject, suffering from chronic inflammatory arthritis associated with Lyme disease bears T-cells reactive to ECGF or ECGF peptides by providing a set of at least one synthesized ECGF Peptide that are predicted to be presented by HLA-DR molecules; stimulating PBMC or the SFMC obtained from the subject with at least one of the ECGF peptides; and measuring T-Cell proliferation *in vitro* or secretion of IFN-γ as a test for T-cell reactivity. The term "subject" as used herein refers to any individual or patient to which the subject methods are performed. Generally the subject is human, although as will be appreciated by those in the art, the subject may be an animal. Thus, other animals, including mammals such as rodents (including mice, rats, hamsters and guinea pigs), cats, dogs, rabbits, farm animals including cows, horses, goats, sheep, pigs, etc., and non human primates (including monkeys, chimpanzees, orangutans and gorillas) are included within the definition of subject.

Another embodiment provides for a method for determining whether a subject, suffering from chronic inflammatory arthritis associated with Lyme disease, has a B cell response to ECGF as determined by the detection of anti-ECGF antibodies in serum, comprising the steps of: (a) providing an isolated antigen, wherein said antigen is ECGF or an ECGF peptide, (b) providing a biological sample from a subject; (c) conducting an immunoassay on said sample utilizing said antigen; wherein said immunoassay detects the presence of antibodies that recognize said ECGF; and (d) determining that the subject contains an antibody against said antigen if the results of the immunoassay indicate that an antibody that recognizes said antigen is present in said sample. For example, the subject suffers from antibiotic-refractory Lyme arthritis or antibiotic-responsive Lyme arthritis. The immunoassay can be an immunoblot or an ELISA, etc. The immunoassay may also include *Borrelia* antigens. The immunoassay may also include other antigens associated with arthritis.

The identification of ECGF autoimmune response in a subject with inflammatory arthritis aids the clinician in diagnosing and/or treating the arthritis indication. For example, presence of ECGF autoantibody (e.g., identified by immunoblot) indicates that autoimmune disease is involved in the subject's inflammation.

### EXAMPLES

### Example 1. ELISPOT assays and synthetic peptides

Enzyme-linked immunosorbent spot (ELISPOT) assays were performed using ELISpot^{plus} for human IFN-γ kits (Mabtech Inc., # 3420-2AW-Plus). Briefly, PBMC collected using Ficoll-Hypaque density centrifugation and stored in liquid nitrogen were thawed quickly and plated in round bottom, 96-well plates (Costar, # 3799) at 2 x 10⁵ per well in 200 µl of complete media (RPMI-1640, 2 mM glutamine, 100 units/ml penicillin 100 µg/ml streptomycin, 10 mM HEPES (all from Invitrogen) and 10% human AB serum (Cellgrow). Peptides were added at a concentration of 1 µM in duplicate wells. Positive and negative controls consisted of 1% PHA (Invitrogen, # 10576-015) and no antigen, respectively. After 5 days at 37°C and 5% CO₂, cells were transferred to ELISPOT plates (Mabtech), previously coated with IFN-γ capture antibody, and incubated overnight. All subsequent steps were performed as detailed in the manufacturer's protocol. Images of wells were captured using ImmunoSpot series 3B analyzer and spots counted using ImmunoSpot 5.0 academic software (Cellular Technology Limited).

The human ECGF has the amino acid sequence:

1 
61 
121 
181 
241 
301 
361 
421 
481 

All peptides were synthesized and HPLC purified at Tufts University Core Facility (Boston, MA). The ECGF peptides sequences were as follows:
ECGF₅₂₋₇₁ ADIRGFVAAVVNGSAQGAQI (SEQ ID NO:2);
ECGF₁₂₃₋₁₃₇ DKVSLVLAPALAACG (SEQ ID NO:3);
ECGF₂₂₀₋₂₃₄ SKKLVEGLSALVVDV (SEQ ID NO:4);
ECGF₂₅₃₋₂₇₄ KTLVGVGASLGLRVAAALTAMD (SEQ ID NO:5);
ECGF₃₀₂₋₃₁₆ LRDLVTTLGGALLWL (SEQ ID NO:6);
ECGF₃₄₀₋₃₅₅ LGRFERMLAAQGVDPG (index case) (SEQ ID NO:1); and
ECGF₃₈₇₋₄₀₁ GTVELVRALPLALVLH (SEQ ID NO:7).

### Example 2. ELISA assays

*Serum anti-ECGF antibody ELISA:* EasyWash ELISA plates (CoStar) were coated with 100 µl of 0.5 µg/ml carrier free, recombinant human PD-ECGF (R&D Systems, #229-PE/CF) dissolved in PBS and incubated overnight at 4°C. All subsequent steps were performed at room temperature with plates on a platform shaker set at 200 rpm. The next day, plates were washed three times with PBST (phosphate buffered saline and 0.05% Tween-20) then incubated with 200 µl of blocking buffer (5% nonfat dry milk in PBST) for 1 hr. Afterwards, wells were washed three times with PBST and 100 µl of each patient's serum sample diluted 1/100 with blocking buffer was added to individual wells and incubated for 1 hr. As a control, serum from eight healthy subjects was added to each plate to be used for inter-plate standardization. After three more washes with PBST, 100 µl goat anti-human IgG conjugated to horseradish peroxidase (KPL # 074-1006) diluted 1:7500 in blocking buffer was added to each well and incubated for 1 hr. Plates were then washed three times with PBST, followed by three times with PBS and incubated with 100 µl of a 1:1 mixture of the substrate 3,3',5,5'-tetramethylbenzidine and 0.01% hydrogen peroxide (TMB substrate reagent kit, # 555214) (BD Biosciences). The reaction was stopped after 3 min with 100 µl of 2N sulfuric acid (LabChem Inc., # LC25790-2). Absorbance values (OD₄₅₀) for each well were determined using a microplate reader (Bio-Rad, model 550).

*Synovial fluid ECGF sandwich ELISA*: EasyWash ELISA plates were coated with 50 µl of the capture antibody, goat anti-human PD-ECGF (Santa Cruz, # SC-9523) diluted in PBS (5 µg/ml) and incubated overnight at 4°C. All subsequent steps were performed at room temperature. The next day, plates were washed three times with PBS and incubated with blocking buffer for 30 min. Afterwards, plates were washed three times with PBS and 100 µl of each patients' synovial fluid sample diluted 1:10 with blocking buffer was added to individual wells and incubated for 2 hr. In order to quantify results, recombinant human PD-ECGF serially diluted with blocking buffer were also added to each plate to generate a standard curve. After washing the plates three times, wells were filled with 150 µl of blocking buffer, gently vortexed and washed again three times with PBS to ensure removal of all unbound proteins. Plates were then incubated with 50 µl of the mouse anti-human PD-ECGF antibody (Santa Cruz, SC-47702) diluted in blocking buffer (5 ng/ml) for 2 hr. Plates were again washed with PBS and 50 µl of the detection antibody, goat anti-mouse IgG conjugated to horse radish peroxidase (Santa Cruz, #SC-2005) diluted in blocking buffer (1:1000) was added to plates and incubated for 1 hr. After plates were washed three times with PBS, 100 µl of TMB was added for ~6 min and then the reaction was stopped with 100 µl of 2N sulfuric acid. Plates were read as described above.

### Example 3. Immunoblotting

Human recombinant PD-ECGF (12 µg) was electrophoresed through a 10% mini-PROTEAN TGX gels (Bio-Rad) then transferred to nitrocellulose membranes. All subsequent steps were performed at room temperature with rocking. Membranes were cut into strips, individually placed into eight channel reservoir liners (Costar, #4878) and incubated for 1 hr in 1.5 ml blocking buffer (5% nonfat dry milk, 0.1% Tween-20 in 20 mM Tris, 500 mM sodium chloride; pH 7.5). Afterwards, strips were washed three times for 1 min intervals with rinse buffer (0.1% Tween-20 in 20 mM Tris, 500 mM sodium chloride; pH 7.5) and each individual strip was incubated for 1 hr with patient's serum diluted 1:100 in blocking buffer. Strips were again washed three times with rinse buffer and incubated for 1 hr with goat anti-human IgG antibody conjugated to alkaline phosphatase (KPL, #4751-1006) diluted 1:2000 in blocking buffer. Strips were washed three times with rinse buffer and another three times with 20 mM Tris, 500 mM sodium chloride; pH 7.5. Bands were visualized by incubation with NBT/BCIP substrate solution (Roche Diagnostics GmbH, # 11681451001) for 3-5 min after which the strips were washed with copious amounts of water to stop the reaction. Bands were considered positive if darker than the pre-determined positive control sample included in each assay.

### Example 4. Immunohistochemical characterization

Synovial tissue biopsies obtained from antibiotic-refractory Lyme arthritis patients after synovectomies were placed in optimal cutting temperature (Tissue-Tek, Sakura, Japan) and stored in liquid nitrogen. Subsequently, 6-8 µm-thick cryosections were cut and stored at -70°C until use. After an initial review by hematoxylin-eosin of the biopsies; sections selected were those in which lining, sublining and subsynovium were present.

The presence of ECGF was assay by immunohistochemital staining performing the immunoperoxidase technique. The sections were fixed in cold acetone for 3 min and air dried. Fixed sections were washed in PBS. Endogenous peroxidase was blocked by incubating the sections with 3% hydrogen peroxide in methanol for 10 min. After rinsing with PBS three times, nonspecific reaction was blocked by incubating sections in 1X power block solution (Biogenex cat. No. HK085-5K) containing 10% normal donkey serum. The sections were then incubated at 4°C overnight with appropriate dilution (3 µg/ml) of anti-rabbit polyclonal PD-ECGF (Abcam Cat. No. ab75920). Negative controls were done using nonspecific rabbit IgG (Sigma) as the primary antibody at the same IgG concentrations. After 5 min rinses with PBS, the sections were incubated with biotinylated anti-rabbit secondary antibody (Biogenex Cat. No.HK3260709) for 40 min at room temperature, rinsed in PBS, and incubated with peroxidase-streptavidin (Biogenex HK320-UK) for 20 min. After three rinses with PBS, the sections were incubated with diamiobenzidine substrate (Biogenex HK130-5K) for up to 10 min. The sections were washed in distilled water and counterstained with Mayer's hemotoxylin, and glycerol-mounted. Microscopic images were obtained with a Nikon eclipse ME6000 microscope using a Nikon digital camera DXM1200C. The intensity of PD-ECGF staining on each synovial tissue regions (lining, sublining and subsynovium) was graded on an arbitrary scale of 0-3: 0 = no ECGF positive stain cells; 1 = few (∼50) positive staining cells; 2 = many (∼50 to 100) positive staining cells; and 3= most (>100) positive cells determined by counting a total of five 200X microscopic fields on for each synovial tissue regions (lining, sublining, and subsynovium); to sample a larger area per section every fifth 200X microscopic field was examined. For illustration purposes, whole-slide imaging of five random biopsies with moderate to intense stain in all synovial regions were scanned using with Mirax Viewer Scan (Carl Zeiss), at 20x/0.8 Plan-Apochromat objective and image was prepared using Mirax viewer software, as shown in Figure 5.

## Claims

1. A method for diagnosing Lyme arthritis in a subject, the method comprising analyzing a biological sample obtained from a subject having Lyme disease for the presence of an endothelial cell growth factor (ECGF) autoantibody by contacting said biological sample with an immunoassay comprising an ECGF antigen and
wherein said biological sample is previously obtained from peripheral blood, serum, synovial fluid, synovial tissue, peripheral blood mononuclear cells (PBMC), or synovial fluid mononuclear cells (SFMC).

2. The method of claim 1, wherein said immunoassay is an ELISA, agglutination test, direct immunofluorescence assay, indirect immunofluorescence assay, or an immunoblot assay, or wherein the immunoassay is a T-cell proliferation assay, optionally wherein the T-cell proliferation assay is a 3H-thymdine incorporation assay, CFSE dilution, or an ELISPOT.

3. The method of claim 1, wherein the immunoassay is a T-cell reactivity assay, optionally wherein the immunoassay comprises measuring secretion of IFN-γ or other cytokines and/or chemokines.

4. The method of any one of the preceding claims, wherein said ECGF antigen is ECGF, or at least one ECGF peptide selected from the group consisting of:
LGRFERMLAAQGVDPG (SEQ ID NO: 1);
ADIRGFVAAVVNGSAQGAQI (SEQ ID NO:2);
DKVSLVLAPALAACG (SEQ ID NO:3);
SKKLVEGLSALVVDV (SEQ ID NO:4);
KTLVGVGASLGLRVAAALTAMD (SEQ ID NO:5);
LRDLVTTLGGALLWL (SEQ ID NO:6); and
GTVELVRALPLALVLH (SEQ ID NO:7).

5. Use of an isolated ECGF antigen as a biomarker for diagnosing Lyme arthritis using an *in vitro* assay, optionally wherein said ECGF antigen is selected from the group consisting of:
ECGF;
LGRFERMLAAQGVDPG (SEQ ID NO: 1);
ADIRGFVAAVVNGSAQGAQI (SEQ ID NO:2);
DKVSLVLAPALAACG (SEQ ID NO:3);
SKKLVEGLSALVVDV (SEQ ID NO:4);
KTLVGVGASLGLRVAAALTAMD (SEQ ID NO:5);
LRDLVTTLGGALLWL (SEQ ID NO:6); and
GTVELVRALPLALVLH (SEQ ID NO:7).

6. An *in vitro* method for determining whether a subject, suffering from chronic inflammatory arthritis associated with Lyme disease, bears T-cells reactive to endothelial cell growth factor (ECGF) or ECGF peptides comprising the steps of: (a) providing a set of synthesized ECGF Peptides that are predicted to be presented by HLA-DR molecules associated with chronic inflammatory arthritis; (b) stimulating peripheral blood mononuclear cells (PBMC) or the synovial fluid mononuclear cells (SFMC) with one of said ECGF Peptides; and (c) measuring T-Cell proliferation in vitro or secretion of IFN-γ into cell culture supernatants as a test for T-cell reactivity.

7. The method of claim 6, wherein the subject suffers from antibiotic-refractive Lyme arthritis, optionally wherein the subject suffers from antibiotic-responsive Lyme arthritis.

8. An *in vitro* method for determining whether a subject, suffering from chronic inflammatory arthritis associated with Lyme disease, contains a B-cell response to ECGF resulting in the production of autoantibodies found in serum or synovial fluid against ECGF, comprising the steps of: (a) providing an isolated antigen, wherein said antigen is ECGF, or an ECGF peptide; (b) providing a biological sample previously obtained from a subject; (c) conducting an immunoassay
on said biological sample utilizing said antigen; wherein said immunoassay detects the presence of antibodies that recognize said ECGF; and (d) determining that the subject contains an antibody against said antigen if the results of the immunoassay indicate that an antibody that recognizes said antigen is present in said biological sample.

9. The method of claim 8, wherein the subject suffers from antibiotic-refractory Lyme arthritis, optionally wherein the subject suffers from antibiotic-responsive Lyme arthritis, or wherein the immunoassay is an enzyme-linked immunosorbent assay (ELISA).

10. A kit for identifying a patient with Lyme arthritis comprising: ECGF or a set of synthesized ECGF peptides, and reagents necessary for conducting an immunoassay, wherein the immunoassay is capable of detecting the presence of an ECGF antibody in a biological sample obtained from a subject having Lyme disease, wherein the antibody is capable of binding to said antigen, and wherein said kit further comprises *Borrelia* antigens.

11. The kit of claim 10, wherein the Lyme arthritis is antibiotic-resistant Lyme arthritis, or wherein the Lyme arthritis is antibiotic-responsive Lyme arthritis, or wherein the immunoassay is an enzyme-linked immunosorbent assay (ELISA), or wherein the immunoassay is a western blot.

## Patentansprüche

1. Verfahren zum Diagnostizieren von Lyme-Arthritis in einem Subjekt, wobei das Verfahren ein Analysieren einer biologischen Probe, welche von einem Subjekt mit Borreliose erhalten wurde, hinsichtlich der Anwesenheit eines Endothelzellen-Wachstumsfaktor-(engl. *endothelial cell growth factor,* ECGF)-Autoantikörpers durch In-Kontakt-bringen der biologischen Probe mit einem Immunassay umfassend ein ECGF-Antigen umfasst und wobei die biologische Probe zuvor aus peripheralem Blut, Serum, Synovialflüssigkeit, Synovialgewebe, peripheren mononuklären Blutzellen (engl. peripheral blood mononuclear cells, PBMC) oder mononukleären Synovialflüssigkeitszellen (engl. *synovial fluid mononuclear cells,* SFMC) erhalten wurde.

2. Verfahren nach Anspruch 1, wobei der Immunassay ein ELISA, Agglutinationstest, direkter Immunfluoreszenzassay, indirekter Immunfluoreszenassay oder ein Immunoblotassay ist, oder wobei der Immunassay ein T-Zell-Proliferationsassay ist, gegebenenfalls wobei der T-Zell-Prolifarationsassay ein 3H-Thymidin-Einbauassay, eine CFSE-Verdünnung oder ein ELISPOT ist.

3. Verfahren nach Anspruch 1, wobei der Immunassay ein T-Zell-Reaktivitätsassay ist, gegebenenfalls wobei der Immunassay ein Messen der Sekretion von IFN-γ oder weiteren Zytokinen und/oder Chemokinen umfasst.

4. Verfahren nach einem der vorherigen Ansprüche, wobei das ECFG-Antigen ECGF ist oder wenigstens ein ECGF-Peptid ausgewählt aus der Gruppe bestehend aus:
LGRFERMLAAQGVDPG (SEQ ID NO: 1);
ADIRGFVAAVVNGSAQGAQI (SEQ ID NO: 2);
DKVSLVLAPALAACG (SEQ ID NO: 3);
SKKLVEGLSALVVDV (SEQ ID NO: 4);
KTLVGVGASLGLRVAAALTAMD (SEQ ID NO: 5);
LRDLVTTLGGALLWL (SEQ ID NO: 6); und
GTVELVRALPLALVLH (SEQ ID NO: 7).

5. Verwendung eines isolierten ECGF-Antigens als ein Biomarker zum Diagnostizieren von Lyme-Arthritis unter Verwendung eines *in-vitro-*Assay*,* gegebenenfalls wobei das ECFG-Antigen ausgewählt ist aus der Gruppe bestehend aus:
ECGF;
LGRFERMLAAQGVDPG (SEQ ID NO: 1);
ADIRGFVAAVVNGSAQGAQI (SEQ ID NO: 2);
DKVSLVLAPALAACG (SEQ ID NO: 3);
SKKLVEGLSALVVDV (SEQ ID NO: 4);
KTLVGVGASLGLRVAAALTAMD (SEQ ID NO: 5);
LRDLVTTLGGALLWL (SEQ ID NO: 6); und
GTVELVRALPLALVLH (SEQ ID NO: 7).

6. in-vitro-Verfahren zum Bestimmen, ob ein Subjekt, welches an chronisch-entzündlicher Arthritis leidet, die mit Borreliose assoziiert ist, T-Zellen aufweist, die gegenüber dem Endothelzellen-Wachstumsfaktor (ECGF) oder ECGF-Peptiden reaktiv sind, umfassend die Schritte: (a) Bereitstellen eines Satzes von synthetisierten ECGF-Peptiden, für die ausgegangen wird, dass sie von HLA-DR-Molekülen präsentiert werden, die mit chronisch-entzündlicher Arthritis assoziiert sind; (b) Stimulieren von peripheren mononukleären Blutzellen (PBMC) oder der mononukleären Synovialflüssigkeitszellen (SFMC) mit einem der ECGF-Peptide; und (c) Messen der T-Zell-Proliferation *in vitro* oder die Sekretion von IFN-γ in die Zellkulturüberstände als ein Test der T-Zell-Reaktivität.

7. Verfahren nach Anspruch 6, wobei das Subjekt an Antibiotika-refraktiver Lyme-Arthritis leidet, gegebenenfalls wobei das Subjekt an Antibiotika-responsiver Lyme-Arthritis leidet.

8. in-vitro-Verfahren zum Bestimmen, ob ein Subjekt, welches an chronisch-entzündlicher Arthritis leidet, die mit Borreliose assoziiert ist, eine B-Zell-Antwort auf ECGF enthält, was zu einer Erzeugung von Autoantikörpern führt, die im Serum oder in Synovialflüssigkeit gegen ECGF gefunden werden, umfassend die Schritte: (a) Bereitstellen eines isolierten Antigens, wobei das Antigen ECGF oder ein ECGF-Peptid ist; (b) Bereitstellen einer biologischen Probe, die zuvor von dem Subjekt erhalten wurde; (c) Durchführen eines Immunassays mit der biologischen Probe unter Verwendung des Antigens, wobei der Immunassay die Anwesenheit von Antikörpern nachweist, die das ECGF erkennen; und (d) Feststellen, dass das Subjekt einen Antikörper gegen das Antigen enthält, falls die Ergebnisse des Immunassay angeben, dass ein Antikörper in der biologischen Probe vorliegt, der das Antigen erkennt.

9. Verfahren nach Anspruch 8, wobei das Subjekt an Antibiotika-refraktorischer Lyme-Arthritis leidet, gegebenenfalls wobei das Subjekt an Antibiotika-responsiver Lyme-Arthritis leidet, oder wobei der Immunassay ein Enzyme Linked Immunosorbent Assay (ELISA) ist.

10. Kit zum dentifizieren eines Patienten mit Lyme-Arthritis umfassend: ECGF oder ein Satz synthetisierter ECGF-Peptide und zur Durchführung eines Immunassays notwendige Reagenzien, wobei der Immunassay in der Lage ist, die Anwesenheit eines ECGF-Antikörpers in einer biologischen Probe nachzuweisen, welche von einem Subjekt mit Borreliose erhalten wurde, wobei der Antikörper in der Lage ist, das Antigen zu binden, und wobei der Kit ferner *Borrelia-*Antigene umfasst.

11. Kit nach Anspruch 10, wobei die Lyme-Arthritis eine Antibiotika-resistente Lyme-Arthritis ist oder wobei die Lyme-Arthritis eine Antibiotika-responsive Lyme-Arthritis ist oder wobei der Immunassay ein Enzyme Linked Immunosorbent Assay (ELISA) ist oder der Immunassay ein Western-Blot ist.

## Revendications

1. Procédé pour le diagnostic de l'arthrite de Lyme chez un sujet, le procédé comprenant l'analyse d'un échantillon biologique obtenu d'un sujet souffrant de la maladie de Lyme pour la présence d'un auto-anticorps dirigé contre le facteur de croissance des cellules endothéliales (ECGF) par la mise en contact dudit échantillon biologique avec un test immunologique comprenant un antigène ECGF et
dans lequel ledit échantillon biologique est obtenu auparavant à partir de sang périphérique, de sérum, de liquide synovial, de tissu synovial, de cellules mononucléées du sang périphérique (CMSP), ou de cellules mononucléées du liquide synovial (CMLS).

2. Procédé selon la revendication 1, dans lequel ledit test immunologique est un test ELISA, un test d'agglutination, un test d'immunofluorescence directe, un test d'immunofluorescence indirecte, ou un test immunoblot, ou dans lequel le test immunologique est un test de prolifération des lymphocytes T, éventuellement dans lequel le test de prolifération des lymphocytes T est un test d'incorporation de 3H-thymidine, la dilution de CFSE, ou un ELISPOT.

3. Procédé selon la revendication 1, dans lequel le test immunologique est un test de réactivité des lymphocytes T, éventuellement dans lequel le test immunologique comprend la mesure de la sécrétion de l'IFN-γ ou d'autres cytokines et/ou chimiokines.

4. Procédé selon l'une quelconque des revendications précédentes, dans lequel ledit antigène ECGF est l'ECGF, ou au moins un peptide de l'ECGF choisi dans le groupe constitué de :
LGRFERMLAAQGVDPG (SEQ ID NO : 1) ;
ADIRGFVAAVVNGSAQGAQI (SEQ ID NO : 2) ;
DKVSLVLAPALAACG (SEQ ID NO : 3) ;
SKKLVEGLSALVVDV (SEQ ID NO : 4) ;
KTLVGVGASLGLRVAAALTAMD (SEQ ID NO : 5) ;
LRDLVTTLGGALLWL (SEQ ID NO : 6) ; et
GTVELVRALPLALVLH (SEQ ID NO : 7).

5. Utilisation d'un antigène ECGF isolé en tant que biomarqueur pour le diagnostic de l'arthrite de Lyme en utilisant un test *in vitro,* éventuellement dans laquelle ledit antigène ECGF est choisi dans le groupe constitué de :
ECGF ;
LGRFERMLAAQGVDPG (SEQ ID NO : 1) ;
ADIRGFVAAVVNGSAQGAQI (SEQ ID NO : 2) ;
DKVSLVLAPALAACG (SEQ ID NO : 3) ;
SKKLVEGLSALVVDV (SEQ ID NO : 4) ;
KTLVGVGASLGLRVAAALTAMD (SEQ ID NO : 5) ;
LRDLVTTLGGALLWL (SEQ ID NO : 6) ; et
GTVELVRALPLALVLH (SEQ ID NO : 7).

6. Procédé *in vitro* pour déterminer si un sujet, souffrant d'une arthrite inflammatoire chronique associée à la maladie de Lyme, porte des lymphocytes T réactifs contre le facteur de croissance des cellules endothéliales (ECGF) ou des peptides de l'ECGF comprenant les étapes de :
(a) fourniture d'un ensemble de peptides de l'ECGF synthétisés qui sont prédits être présentés par des molécules HLA-DR associées à l'arthrite inflammatoire chronique ;
(b) stimulation des cellules mononucléées du sang périphérique (CMSP) ou des cellules mononucléées du liquide synovial (CMLS) par l'un desdits peptides de l'ECGF ; et
(c) mesure de la prolifération de lymphocytes T *in vitro* ou de la sécrétion de l'IFN-γ dans les surnageants des cultures cellulaires comme test pour la réactivité des lymphocytes T.

7. Procédé selon la revendication 6, dans lequel le sujet souffre de l'arthrite de Lyme réfractaire aux antibiotiques, éventuellement dans lequel le sujet souffre de l'arthrite de Lyme sensible aux antibiotiques.

8. Procédé *in vitro* pour déterminer si un sujet, souffrant de l'arthrite inflammatoire chronique associée à la maladie de Lyme, contient une réponse des lymphocytes B contre l'ECGF entraînant la production d'auto-anticorps trouvés dans le sérum ou le liquide synovial contre l'ECGF, comprenant les étapes de :
(a) fourniture d'un antigène isolé, dans lequel ledit antigène est l'ECGF, ou un peptide de l'ECGF ;
(b) fourniture d'un échantillon biologique obtenu auparavant à partir d'un sujet ;
(c) conduite d'un test immunologique sur ledit échantillon biologique en utilisant ledit antigène ; dans lequel ledit test immunologique détecte la présence d'anticorps qui reconnaissent ledit ECGF ; et
(d) détermination que le sujet contient un anticorps dirigé contre ledit antigène si les résultats du test immunologique indiquent qu'un anticorps qui reconnaît ledit antigène est présent dans ledit échantillon biologique.

9. Procédé selon la revendication 8, dans lequel le sujet souffre de l'arthrite de Lyme réfractaire aux antibiotiques, éventuellement dans lequel le sujet souffre de l'arthrite de Lyme sensible aux antibiotiques, ou dans lequel le test immunologique est un test ELISA (enzyme-linked immunosorbent assay).

10. Kit pour identifier un patient souffrant de l'arthrite de Lyme comprenant : l'ECGF ou un ensemble de peptides de l'ECGF synthétisés, et des réactifs nécessaires pour conduire un test immunologique, dans lequel le test immunologique est capable de détecter la présence d'un anticorps dirigé contre l'ECGF dans un échantillon biologique obtenu d'un sujet souffrant de la maladie de Lyme, dans lequel l'anticorps est capable de se lier au dit antigène, et dans lequel ledit kit comprend en outre des antigènes de *Borrelia*.

11. Kit selon la revendication 10, dans lequel l'arthrite de Lyme est l'arthrite de Lyme résistante aux antibiotiques, ou dans lequel l'arthrite de Lyme est l'arthrite de Lyme sensible aux antibiotiques, ou dans lequel le test immunologique est un test ELISA (enzyme-linked immunosorbent assay), ou dans lequel le test immunologique est un Western blot.
